# EUROPEAN PATENT APPLICATION

(11) **EP 4 534 562 A1**
(43) Date of publication of application: **09.04.2025**
(21) Application number: 23910941.6
(22) Date of filing: 29.12.2023
(51) Int. Cl.: C07K 16/46, C07K 16/22, A61K 39/395, A61P 35/00, C12N 15/13

(54) **BISPECIFIC ANTIBODY AND APPLICATION THEREOF**

(30) Priority: 30.12.2022 CN 202211722833
(71) Applicant: Remegen (Shanghai) Co., Ltd., Shanghai 201210 (CN); RemeGen Co., Ltd., Shandong 264006 (CN)
(72) Inventor: LI, Dong, Shanghai 201210 (CN); FANG, Jianmin, Shandong 264006 (CN); YUAN, Mei, Shanghai 201210 (CN); CHEN, Shanshan, Shanghai 201210 (CN); WANG, Sisi, Shanghai 201210 (CN); XIN, Yinghao, Shandong 264006 (CN); LI, Yuanhao, Shandong 264006 (CN); ZHAO, Guorui, Shandong 264006 (CN); MA, Xinting, Shandong 264006 (CN)
(74) Representative: HGF
(86) International application number: PCT/CN2023/143288
(87) International publication number: WO 2024/141027

(57) **Abstract**

A VEGF-targeting VHH, a bispecific antibody targeting PD-1 and VEGF developed on the basis of the VHH, and an application thereof. The VHH has high stability, and has a plurality of excellent effects, such as ease of expression and purification. The constructed bispecific antibody targeting PD-1 and VEGF has high stability, can target enrichment in a high-expression VEGF tumor region, has good medicinal efficacy and safety, and has excellent treatment potential.

## Description

### FIELD

The present disclosure relates to the field of antibodies, and in particular to a bispecific antibody targeting PD-1 and VEGF and use thereof.

### BACKGROUND

Programmed death receptor-1 (PD-1) belongs to the immunoglobulin CD28/B7 superfamily and is an immunosuppressive receptor. The binding of PD-1 to programmed death-ligand 1 (PD-L1) results in the phosphorylation of phosphatidylinositol-3-kinase, the further activation of protein kinase B, the activation of stimulatory T cell signaling pathways, glucose metabolism, and the secretion of interferons, which leads to the block of the downstream signals of T cell activation, thereby effectively inhibiting the transcription of T cells and, in turn, inhibiting the immune function of T cells. This process plays a pivotal role in the regulation of negative immune responses. The broad-spectrum anti-tumor efficacy of PD-1 antibodies has led to the launch of numerous blockbuster drugs, such as O drug (trade name: Opdivo; generic name: Nivolumab), which was approved in the United States on December 22, 2014. Its initial indication was melanoma, and it was subsequently approved in the United States for indications including non-small cell lung cancer, renal cell carcinoma, Hodgkin lymphoma, head and neck squamous cell carcinoma, colorectal cancer, hepatocellular carcinoma, urothelial cancer, colorectal cancer, hepatocellular carcinoma, esophageal cancer, esophageal squamous cell carcinoma, pleural mesothelioma, esophageal adenocarcinoma, gastroesophageal junction cancer, gastric cancer, etc. However, an analysis of existing clinical data including 28,304 patient records from 160 clinical trials (Reference 1: Efficacy of PD-1/PD-L1 blockade monotherapy in clinical trials, Bin Zhao, Hong Zhao, et al., Ther Adv Med Oncol. 2020, Vol. 12, pp. 1-22.) indicates that a total of 4,747 patients out of 22,165 patients who received PD-1/PD-L1 monotherapy generally showed a treatment effect [objective response rate (ORR), 20.21%; 95% confidence interval (CI), 18.34-22.15%]. A total of 862 complete responses (CR) were observed among 19,418 cancer patients. The overall incidence of CR was 3.85% (95% CI, 3.06-4.73%). The ORR is significantly different in different cancer types and PD-L1 expression status. That is to say, the efficacy of PD-1/PD-L1 monotherapy varies considerably between different cancer types and PD-L1 expression status. Consequently, there is still a significant clinical need that remains unmet.

Vascular endothelial growth factor (VEGF), also known as vascular permeability factor (VPF), is a highly specific factor that promotes vascular permeability, extracellular matrix degeneration, endothelial cell migration, proliferation, angiogenesis, etc. The data indicate that VEGF plays a significant role in pathological angiogenesis and can induce the occurrence and progression of various pathologies, including tumor growth and metastasis, macular degeneration, diabetic retinopathy, inflammatory processes (such as rheumatoid arthritis), ischemic processes (myocardial ischemia), preeclampsia, and so forth. (Reference 2: Molecular and functional diversity of vascular endothelial growth factors. Yamazaki Y., Morita T., Mol. Divers. November 2006, Vol. 10, No. 4, pp. 515-527.). The high-affinity receptor that specifically binds to vascular endothelial growth factor is called vascular endothelial growth factor receptor (VEGFR). It is a tyrosine kinase receptor that plays a key role in many signal transduction pathways essential for angiogenesis and cell migration. VEGF binds to VEGFR to initiate the signal cascade and stimulates angiogenesis. Therefore, VEGF/NEGFR is a very important target because they are almost exclusively expressed in endothelial cells and are highly upregulated in many tumor endothelial types. Currently, the use of anti-VEGF/VEGFR therapy to block angiogenesis in cancer or other pathological processes is considered extremely important. The inhibition of vascular endothelial growth factor/vascular endothelial growth factor receptor represents the current mainstay of treatment for certain cancers, including renal cell carcinoma, hepatocellular carcinoma, etc.

At present, numerous clinical trials are investigating the safety and efficacy of vascular endothelial growth factor/vascular endothelial growth factor receptor in combination with immune checkpoint inhibitors in cancer treatment. Some preliminary studies have shown that, compared to single VEGFR inhibition or immunosuppression, the combined use of immune checkpoint inhibitors and VEGFR inhibitors may result in superior therapeutic outcomes in patients
(https://www.obroncology.com/article/combining-immune-checkpoint-and-vegf-inhibitors-impro ves-survival-in-pretreated-nsclc). However, the potential risks associated with this favourable outcome remain uncertain (Reference 3: Combining Immune Checkpoint and VEGFR Inhibition in Favorable Risk and Elderly Patients with Metastatic Renal Cell Carcinoma. Varkaris A, Xu W, Davis RB, Healy B, McDermott DF. Clin Genitourin Cancer. June 2020, Vol. 18, No. 3, pp. 179-184.). In some clinical trials of VEGFR/ICI dual blockade (e.g., pembrolizumab/pazopanib combination group, nivolumab/sunitinib combination group, nivolumab/pazopanib combination group, etc.), the results indicated promising therapeutic effects in the early stages of clinical research (Reference 4: A Phase I/II Study to Assess the Safety and Efficacy of Pazopanib and Pembrolizumab Combination Therapy in Patients with Advanced Renal Cell Carcinoma. Chowdhury S, Infante JR, et al. Clin Genitourin Cancer. 2021 rh October, Vol. 19, No. 5, pp. 434-446; Reference 5: Nivolumab (anti-PD-1; BMS-936558, ONO-4538) in combination with sunitinib or pazopanib in patients (pts) with metastatic renal cell carcinoma (mRCC). Amin, A. et al., Journal of Clinical Oncology. 32, (suppl.), May 20, 2014, Abstract 5010). However, severe toxicity was observed in the subsequent high-dose group. 73% and 60% of patients experienced grade 3 or more severe treatment-related toxicity in the nivolumab/sunitinib combination group and nivolumab/pazopanib combination group, respectively. Furthermore, 23% of patients in the nivolumab/sunitinib combination group and 20% of patients in the nivolumab/pazopanib group discontinued treatment due to adverse events. Similarly, high-dose-related hepatotoxicity occurred in the pembrolizumab/pazopanib combination group. Therefore, these combinations were considered unsuitable for testing in phase 3 trials and were thus terminated. In addition, positive efficacy data were also reported in other clinical trials of VEGFR/ICI dual blockade, such as the phase III clinical trial of bevacizumab combined with atezolizumab (Reference 6: IMmotion151: a randomized phase III study of atezolizumab plus bevacizumab vs sunitinib in untreated metastatic renal cell carcinoma (mRCC), Motzer, RJ et al. Journal of Clinical Oncology. 36, abstr. 578 (2018).). In this trial, the remission rate of patients was improved. However, according to the MSKCC standards, only 20% of the intention-to-treat patients in this clinical trial had a favorable risk. The main reason was that the combination therapy also increased the toxic side effects of patients.

In addition to the exploration of combined therapy, there are also a number of bispecific antibodies targeting VEGF/VEGF receptor and PD-1 that are still under difficult exploration, such as AK112 developed by Akeso Biomedical, Inc., Zhongshan (see Chinese patent application CN109053895). Nevertheless, to date, no bispecific antibodies targeting VEGF/VEGF receptor and immune checkpoints have been approved for marketing worldwide. There remain significant clinical needs and development challenges.

### SUMMARY

Through inventive research, the inventors develop a bispecific antibody targeting PD-1 and VEGF, wherein a variable domain of heavy chain of heavy-chain antibody (VHH) targeting VEGF, obtained after multiple rounds of screening and optimization, as a modular component of the binding functional region, is combined with a PD-1 antibody. The bispecific antibody comprises:
(a) a first binding functional region targeting PD-1; and
(b) a second binding functional region targeting VEGF;
wherein:
the first binding functional region targeting PD-1 is an anti-PD-1 antibody (Ab) or an antigen-binding fragment thereof (Ab'); and
the second binding functional region targeting VEGF comprises a VHH domain of complementarity-determining regions (CDRs) 1-3 defined by the following amino acid sequences:

| | |
|---|---|
| CDR1 | SEQ ID NO: 1 |
| CDR2 | SEQ ID NO: 2 with or without one or two amino acid mutations |
| CDR3 | SEQ ID NO: 3 |

Further, the amino acid mutation in CDR2 is at position 58 and/or position 65.

Further, the amino acid mutation in CDR2 is N58Y and/or D65G mutation.

Preferably, the amino acid mutation in CDR2 is N58Y and D65G mutations.

Preferably, the amino acid sequence of CDR2 with mutation is set forth in SEQ ID NO: 24.

Preferably, the combination of CDRs 1-3 of the VHH domain of the second binding functional region targeting VEGF is:

| | |
|---|---|
| CDR1 | SEQ ID NO: 1 |
| CDR2 | SEQ ID NO: 2 |
| CDR3 | SEQ ID NO: 3 |

or

| | |
|---|---|
| CDR1 | SEQ ID NO: 1 |
| CDR2 | SEQ ID NO: 24 |
| CDR3 | SEQ ID NO: 3 |

Further, the VHH domain is a humanized VHH domain.

Further, the second binding functional region targeting VEGF further comprises:
1) a first framework region domain FR1 comprising an amino acid sequence set forth in SEQ ID NO: 4 with or without one or two amino acid mutations; and/or
2) a second framework region domain FR2 comprising an amino acid sequence set forth in SEQ ID NO: 5 with or without one amino acid mutation; and/or
3) a third framework region domain FR3 comprising an amino acid sequence set forth in SEQ ID NO: 6 with or without 1-5 amino acid mutations; and/or
4) a fourth framework region domain FR4 comprising an amino acid sequence set forth in SEQ ID NO: 7 with or without one amino acid mutation.

Further, the one or two amino acid mutations in FR1 are at position 1 and/or position 5; preferably Q1E and/or Q5L mutations.

Further, the one amino acid mutation in FR2 is at position 49; preferably A49S mutation.

Further, the 1-5 amino acid mutations in FR3 are at positions selected from the group consisting of positions 74, 82B, 83, 84, 89, and a combination thereof; preferably, the 1-5 amino acid mutations are selected from the group consisting of D74S, V(82B)S, K83R, P84A, M89V and a combination thereof.

Further, the one amino acid mutation in FR4 is at position 108; preferably Q108L.

Further, the second binding functional region targeting VEGF further comprises:
1) a first framework region domain FR1 comprising an amino acid sequence set forth in SEQ ID NO: 4 or SEQ ID NO: 9; and/or
2) a second framework region domain FR2 comprising an amino acid sequence set forth in SEQ ID NO: 5 or SEQ ID NO: 10; and/or
3) a third framework region domain FR3 comprising an amino acid sequence set forth in SEQ ID NO: 6 or SEQ ID NO: 11; and/or
4) a fourth framework region domain FR4 comprising an amino acid sequence set forth in SEQ ID NO: 7 or SEQ ID NO: 12.

Further, the second binding functional region targeting VEGF comprises a framework region combination defined by the following amino acid sequences:

| | |
|---|---|
| FR1 | SEQ ID NO: 4 |
| FR2 | SEQ ID NO: 5 |
| FR3 | SEQ ID NO: 6 |
| FR4 | SEQ ID NO: 7 |

or

| | |
|---|---|
| FR1 | SEQ ID NO: 9 |
| FR2 | SEQ ID NO: 10 |
| FR3 | SEQ ID NO: 11 |
| FR4 | SEQ ID NO: 12 |

or

| | |
|---|---|
| FR1 | SEQ ID NO: 9 |
| FR2 | SEQ ID NO: 5 |
| FR3 | SEQ ID NO: 11 |
| FR4 | SEQ ID NO: 12 |

Further, the second binding functional region targeting VEGF comprises an amino acid sequence set forth in SEQ ID NO: 8 with or without 1-11 (1, 2, 3, 4, 5, 6, 7, 8, 9, 10 or 11) amino acid mutations. Further, the amino acid mutation is at a position selected from the group consisting of positions 1, 5, 49, 58, 65, 74, 82B, 83, 84, 89, 108, and a combination thereof. Further, the amino acid mutation is selected from the group consisting of Q1E, Q5L, A49S, N58Y, D65G, D74S, V(82B)S, K83R, P84A, M89V, Q108L and a combination thereof.

Further preferably, the amino acid mutation is selected from the following mutation combinations:

| | |
|---|---|
| Mutation | Q1E+Q5L+A49S+N58Y+D65G+D74S+V(82B)S+K83R+P84A+M89V+Q108L |
| combination 1 | |
| Mutation combination 2 | Q1E+Q5L+N58Y+D65G+D74S+V(82B)S+K83R+P84A+M89V+Q108L |

Further preferably, the second binding functional region targeting VEGF comprises an amino acid sequence set forth in SEQ ID NO: 8, SEQ ID NO: 13 or SEQ ID NO: 14.

**Table 1 Sequences of each structural region of Strain #6 VHH (Kabat system)**

| | |
|---|---|
| CDR1 (SEQ ID NO: 1) | TSTMS |
| CDR2 (SEQ ID NO: 2) | FITSAGATTNYADSVKD |
| CDR3 (SEQ ID NO: 3) | LVTLWNVY |
| FR1 (SEQ ID NO: 4) | QVQLQESGGGLVQPGGSLRLSCAASGFTFS |
| FR2 (SEQ ID NO: 5) | WYRQAPGKERELVA |
| FR3 (SEQ ID NO: 6) | RFTMSRDNDKNTVYLQMNVLKPEDTAMYYCRA |
| FR4 (SEQ ID NO: 7) | WGQGTQVTVSS |
| Strain #6 VHH (SEQ ID NO: 8) | QVQLQESGGGLVQPGGSLRLSCAASGFTFSTSTMSWYRQAPGKE RELVAFITSAGATTNYADSVKDRFTMSRDNDKNTVYLQMNVLKP EDTAMYYCRALVTLWNVYWGQGTQVTVSS |

**Table 2 Sequences of each structural region of Strain #48 VHH (Kabat system)**

| | |
|---|---|
| CDR1 (SEQ ID NO: 1) | TSTMS |
| CDR2 (SEQ ID NO: 24) | FITSAGATTYYADSVKG |
| CDR3 (SEQ ID NO: 3) | LVTLWNVY |
| FR1 (SEQ ID NO: 9) | EVQLLESGGGLVQPGGSLRLSCAASGFTFS |
| FR2 (SEQ ID NO: 10) | WYRQAPGKERELVS |
| FR3 (SEQ ID NO: 11) | RFTMSRDNSKNTVYLQMNSLRAEDTAVYYCRA |
| FR4 (SEQ ID NO: 12) | WGQGTLVTVSS |
| Strain #48 VHH (SEQ ID NO: 13) | EVQLLESGGGLVQPGGSLRLSCAASGFTFSTSTMSWYRQAPGKE RELVSFITSAGATTYYADSVKGRFTMSRDNSKNTVYLQMNSLRA EDTAVYYCRALVTLWNVYWGQGTLVTVSS |

**Table 3 Sequences of each structural region of Strain #49 VHH (Kabat system)**

| | |
|---|---|
| CDR1 (SEQ ID NO: 1) | TSTMS |
| CDR2 (SEQ ID NO: 24) | FITSAGATTYYADSVKG |
| CDR3 (SEQ ID NO: 3) | LVTLWNVY |
| FR1 (SEQ ID NO: 9) | EVQLLESGGGLVQPGGSLRLSCAASGFTFS |
| FR2 (SEQ ID NO: 5) | WYRQAPGKERELVA |
| FR3 (SEQ ID NO: 11) | RFTMSRDNSKNTVYLQMNSLRAEDTAVYYCRA |
| FR4 (SEQ ID NO: 12) | WGQGTLVTVSS |
| Strain #49 VHH ( SEQ ID NO: 14) | EVQLLESGGGLVQPGGSLRLSCAASGFTFSTSTMSWYRQAPGKE RELVAFITSAGATTYYADSVKGRFTMSRDNSKNTVYLQMNSLRA EDTAVYYCRALVTLWNVYWGQGTLVTVSS |

Further, the anti-PD-1 antibody (Ab) or the antigen-binding fragment thereof (Ab') is selected from the group consisting of a humanized antibody or an antigen-binding fragment thereof, a chimeric antibody or an antigen-binding fragment thereof, and a human antibody or an antigen-binding fragment thereof.

Further, the anti-PD-1 antibody (Ab) or the antigen-binding fragment thereof (Ab') is an IgG antibody.

Further, the anti-PD-1 antibody (Ab) or the antigen-binding fragment thereof (Ab') is IgG1, IgG2, or IgG4.

Further, the first binding functional region targeting PD-1 comprises an amino acid sequence of light chain and/or heavy chain CDRs identical to that of nivolumab (Opdivo) or pembrolizumab (Keytruda).

Further, the first binding functional region targeting PD-1 comprises an amino acid sequence of heavy chain variable region identical to nivolumab (Opdivo) or pembrolizumab (Keytruda).

Further, the first binding functional region targeting PD-1 comprises an amino acid sequence of light chain variable region identical to nivolumab (Opdivo) or pembrolizumab (Keytruda).

Further, the first binding functional region targeting PD-1 comprises a heavy chain moiety, wherein the heavy chain moiety further comprises a domain selected from the group consisting of a heavy chain constant region CH1 (CH1), a hinge region (Hinge), a heavy chain constant region CH2 (CH2), a heavy chain constant region CH3 (CH3), and a combination thereof.

Further, the first binding functional region targeting PD-1 further comprises a light chain constant region (CL).

Further, the anti-PD-1 antibody comprises a heavy chain moiety and a light chain moiety, wherein the heavy chain moiety comprises, from N-terminus to C-terminus, a heavy chain variable region (VH), a heavy chain constant region CH1 (CH1), a hinge region (Hinge), a heavy chain constant region CH2 (CH2), and a heavy chain constant region CH3 (CH3), and the light chain moiety comprises, from N-terminus to C-terminus, a light chain variable region (VL) and a light chain constant region (CL).

Further, the Fc domain (i.e., the hinge region, the heavy chain constant region CH2, and the heavy chain constant region CH3) of the anti-PD-1 antibody is an IgG1 Fc domain or a silent IgG1 mutation thereof.

Further, the Fc domain comprises an amino acid sequence set forth in SEQ ID NO: 15 or at least 90%, 91%, 92%, 93%, 94%, 95%, 96%, 97%, 98%, 99% or more identical to an amino acid sequence set forth in SEQ ID NO: 15.

**Table 4 Amino acid sequence of Fc domain**

| | |
|---|---|
| SEQ ID NO: 15 | EPKSCDKTHT CPPCPAPEAE GAPSVFLFPP KPKDTLMISR TPEVTCVVVD VSHEDPEVKF 60 NWYVDGVEVH NAKTKPREEQ YNSTYRVVSV LTVLHQDWLN GKEYKCKVSN KALPSSIEKT 120 ISKAKGQPRE PQVYTLPPSR EEMTKNQVSL TCLVKGFYPS DIAVEWESNG QPENNYKTTP 180 PVLDSDGSFF LYSKLTVDKS RWQQGNVFSC SVMHEALHNH YTQKSLSLSP G 231 |

Further, the first binding functional region targeting PD-1 has a light chain and a heavy chain combination selected from the following amino acid sequences:
1) a heavy chain comprising an amino acid sequence set forth in SEQ ID NO: 16, and/or a light chain comprising an amino acid sequence set forth in SEQ ID NO: 17; or
2) a heavy chain comprising an amino acid sequence set forth in SEQ ID NO: 18, and/or a light chain comprising an amino acid sequence set forth in SEQ ID NO: 19.

**Table 5 Preferred amino acid sequences of the first binding functional region**

| | |
|---|---|
| SEQ ID NO: 16 | QVQLVESGGG VVQPGRSLRL DCKASGITFS NSGMHWVRQA PGKGLEWVAV IWYDGSKRYY 60 ADSVKGRFTI SRDNSKNTLF LQMNSLRAED TAVYYCATND DYWGQGTLVT VSSASTKGPS 120 VFPLAPSSKS TSGGTAALGC LVKDYFPEPV TVSWNSGALT SGVHTFPAVL QSSGLYSLSS 180 VVTVPSSSLG TQTYICNVNH KPSNTKVDKK VEPKSCDKTH TCPPCPAPEA EGAPSVFLFP 240 PKPKDTLMIS RTPEVTCVVV DVSHEDPEVK FNWYVDGVEV HNAKTKPREE QYNSTYRVVS 300 VLTVLHQDWL NGKEYKCKVS NKALPSSIEK TISKAKGQPR EPQVYTLPPS REEMTKNQVS 360 LTCLVKGFYP SDIAVEWESN GQPENNYKTT PPVLDSDGSF FLYSKLTVDK SRWQQGNVFS 420 CSVMHEALHN HYTQKSLSLS PG 442 |
| SEQ ID NO: 17 | EIVLTQSPAT LSLSPGERAT LSCRASQSVS SYLAWYQQKP GQAPRLLIYD ASNRATGIPA 60 RFSGSGSGTD FTLTISSLEP EDFAVYYCQQ SSNWPRTFGQ GTKVEIKRTV AAPSVFIFPP 120 SDEQLKSGTA SVVCLLNNFY PREAKVQWKV DNALQSGNSQ ESVTEQDSKD STYSLSSTLT 180 LSKADYEKHK VYACEVTHQG LSSPVTKSFN RGEC 214 |
| SEQ ID NO: 18 | QVQLVQSGVE VKKPGASVKV SCKASGYTFT NYYMYWVRQA PGQGLEWMGG INPSNGGTNF 60 NEKFKNRVTL TTDSSTTTAY MELKSLQFDD TAVYYCARRD YRFDMGFDYW GQGTTVTVSS 120 ASTKGPSVFP LAPSSKSTSG GTAALGCLVK DYFPEPVTVS WNSGALTSGV HTFPAVLQSS 180 GLYSLSSVVT VPSSSLGTQT YICNVNHKPS NTKVDKKVEP KSCDKTHTCP PCPAPEAEGA 240 PSVFLFPPKP KDTLMISRTP EVTCVVVDVS HEDPEVKFNW YVDGVEVHNA KTKPREEQYN 300 STYRVVSVLT VLHQDWLNGK EYKCKVSNKA LPSSIEKTIS KAKGQPREPQ VYTLPPSREE 360 MTKNQVSLTC LVKGFYPSDI AVEWESNGQP ENNYKTTPPV LDSDGSFFLY SKLTVDKSRW 420 QQGNVFSCSV MHEALHNHYT QKSLSLSPG 449 |
| SEQ ID NO: 19 | EIVLTQSPAT LSLSPGERAT LSCRASKGVS TSGYSYLHWY QQKPGQAPRL LIYLASYLES 60 GVPARFSGSG SGTDFTLTIS SLEPEDFAVY YCQHSRDLPL TFGGGTKVEI KRTVAAPSVF 120 IFPPSDEQLK SGTASVVCLL NNFYPREAKV QWKVDNALQS GNSQESVTEQ DSKDSTYSLS 180 STLTLSKADY EKHKVYACEV THQGLSSPVT KSFNRGEC 218 |

Further, the second binding functional region is linked to the C-terminus or N-terminus of the first binding functional region.

Further, the VHH domain is directly or via a peptide linker linked to the C-terminus or N-terminus of the heavy chain moiety of the antibody (Ab) or the antigen-binding fragment thereof (Ab').

Further, the peptide linker is a flexible peptide linker.

Further, the peptide linker comprises one or more amino acids.

Further, the peptide linker comprises at least 5 amino acids.

Preferably, the peptide linker comprises an amino acid sequence of (GGGGS)ₙ, wherein n is 1, 2, 3 or 4.

Further, the bispecific antibody is a bivalent, trivalent or tetravalent bispecific antibody.

Further, the bispecific antibody comprises a combination of a heavy chain set forth in SEQ ID NO: 20 and a light chain set forth in SEQ ID NO: 21, or comprises a combination of a heavy chain set forth in SEQ ID NO: 22 and a light chain set forth in SEQ ID NO: 23.

**Table 6 Preferred amino acid sequences of bispecific antibodies**

| |
|---|
| #10 |
| Heavy chain (SEQ ID NO: 20): |
| QVQLVESGGG VVQPGRSLRL DCKASGITFS NSGMHWVRQA PGKGLEWVAV IWYDGSKRYY 60 ADSVKGRFTI SRDNSKNTLF LQMNSLRAED TAVYYCATND DYWGQGTLVT VSSASTKGPS 120 VFPLAPSSKS TSGGTAALGC LVKDYFPEPV TVSWNSGALT SGVHTFPAVL QSSGLYSLSS 180 VVTVPSSSLG TQTYICNVNH KPSNTKVDKK VEPKSCDKTH TCPPCPAPEA EGAPSVFLFP 240 PKPKDTLMIS RTPEVTCVVV DVSHEDPEVK FNWYVDGVEV HNAKTKPREE QYNSTYRVVS 300 VLTVLHQDWL NGKEYKCKVS NKALPSSIEK TISKAKGQPR EPQVYTLPPS REEMTKNQVS 360 LTCLVKGFYP SDIAVEWESN GQPENNYKTT PPVLDSDGSF FLYSKLTVDK SRWQQGNVFS 420 CSVMHEALHN HYTQKSLSLS PGEVQLLESG GGLVQPGGSL RLSCAASGFT FSTSTMSWYR 480 QAPGKERELV SFITSAGATT YYADSVKGRF TMSRDNSKNT VYLQMNSLRA EDTAVYYCRA 540 LVTLWNVYWG QGTLVTVSS 559 |
| Light chain (SEQ ID NO: 21): |
| EIVLTQSPAT LSLSPGERAT LSCRASQSVS SYLAWYQQKP GQAPRLLIYD ASNRATGIPA 60 RFSGSGSGTD FTLTISSLEP EDFAVYYCQQ SSNWPRTFGQ GTKVEIKRTV AAPSVFIFPP 120 SDEQLKSGTA SVVCLLNNFY PREAKVQWKV DNALQSGNSQ ESVTEQDSKD STYSLSSTLT 180 LSKADYEKHK VYACEVTHQG LSSPVTKSFN RGEC 214 |
| #16 |
| Heavy chain (SEQ ID NO: 22): |
| QVQLVQSGVE VKKPGASVKV SCKASGYTFT NYYMYWVRQA PGQGLEWMGG INPSNGGTNF 60 NEKFKNRVTL TTDSSTTTAY MELKSLQFDD TAVYYCARRD YRFDMGFDYW GQGTTVTVSS 120 ASTKGPSVFP LAPSSKSTSG GTAALGCLVK DYFPEPVTVS WNSGALTSGV HTFPAVLQSS 180 GLYSLSSVVT VPSSSLGTQT YICNVNHKPS NTKVDKKVEP KSCDKTHTCP PCPAPEAEGA 240 PSVFLFPPKP KDTLMISRTP EVTCVVVDVS HEDPEVKFNW YVDGVEVHNA KTKPREEQYN 300 STYRVVSVLT VLHQDWLNGK EYKCKVSNKA LPSSIEKTIS KAKGQPREPQ VYTLPPSREE 360 MTKNQVSLTC LVKGFYPSDI AVEWESNGQP ENNYKTTPPV LDSDGSFFLY SKLTVDKSRW 420 QQGNVFSCSV MHEALHNHYT QKSLSLSPGE VQLLESGGGL VQPGGSLRLS CAASGFTFST 480 STMSWYRQAP GKERELVSFI TSAGATTYYA DSVKGRFTMS RDNSKNTVYL QMNSLRAEDT 540 AVYYCRALVT LWNVYWGQGT LVTVSS 566 |
| Light chain (SEQ ID NO: 23): |
| EIVLTQSPAT LSLSPGERAT LSCRASKGVS TSGYSYLHWY QQKPGQAPRL LIYLASYLES 60 GVPARFSGSG SGTDFTLTIS SLEPEDFAVY YCQHSRDLPL TFGGGTKVEI KRTVAAPSVF 120 IFPPSDEQLK SGTASVVCLL NNFYPREAKV QWKVDNALQS GNSQESVTEQ DSKDSTYSLS 180 STLTLSKADY EKHKVYACEV THQGLSSPVT KSFNRGEC 218 |

The present disclosure further relates to a VHH domain targeting VEGF, wherein the VHH domain comprises CDRs 1-3 defined by the following amino acid sequences:

| | |
|---|---|
| CDR1 | SEQ ID NO: 1 |
| CDR2 | SEQ ID NO: 2 with or without one or two amino acid mutations |
| CDR3 | SEQ ID NO: 3 |

Further, the amino acid mutation in CDR2 is at position 58 and/or position 65.

Further, the amino acid mutation in CDR2 is N58Y and/or D65G mutation.

Further, the VHH domain is a humanized VHH domain.

Further, the VHH domain further comprises:
1) a first framework region domain FR1 comprising an amino acid sequence set forth in SEQ ID NO: 4 with or without one or two amino acid mutations; and/or
2) a second framework region domain FR2 comprising an amino acid sequence set forth in SEQ ID NO: 5 with or without one amino acid mutation; and/or
3) a third framework region domain FR3 comprising an amino acid sequence set forth in SEQ ID NO: 6 with or without 1-5 amino acid mutations; and/or
4) a fourth framework region domain FR4 comprising an amino acid sequence set forth in SEQ ID NO: 7 with or without one amino acid mutation.

Further, the one or two amino acid mutations in FR1 are at position 1 and/or position 5; preferably Q1E and/or Q5L mutations.

Further, the one amino acid mutation in FR2 is at position 49; preferably A49S mutation.

Further, the 1-5 amino acid mutations in FR3 are at positions selected from the group consisting of positions 74, 82B, 83, 84, 89, and a combination thereof; preferably, the 1-5 amino acid mutations are selected from the group consisting of D74S, V(82B)S, K83R, P84A, M89V and a combination thereof.

Further, the one amino acid mutation in FR4 is at position 108; preferably Q108L.

Further, the VHH domain targeting VEGF further comprises:
1) a first framework region domain FR1 comprising an amino acid sequence set forth in SEQ ID NO: 4 or SEQ ID NO: 9; and/or
2) a second framework region domain FR2 comprising an amino acid sequence set forth in SEQ ID NO: 5 or SEQ ID NO: 10; and/or
3) a third framework region domain FR3 comprising an amino acid sequence set forth in SEQ ID NO: 6 or SEQ ID NO: 11; and/or
4) a fourth framework region domain FR4 comprising an amino acid sequence set forth in SEQ ID NO: 7 or SEQ ID NO: 12.

Further, the VHH domain comprises a framework region combination defined by the following amino acid sequences:

| | |
|---|---|
| FR1 | SEQ ID NO: 4 |
| FR2 | SEQ ID NO: 5 |
| FR3 | SEQ ID NO: 6 |
| FR4 | SEQ ID NO: 7 |

or

| | |
|---|---|
| FR1 | SEQ ID NO: 9 |
| FR2 | SEQ ID NO: 10 |
| FR3 | SEQ ID NO: 11 |
| FR4 | SEQ ID NO: 12 |

or

| | |
|---|---|
| FR1 | SEQ ID NO: 9 |
| FR2 | SEQ ID NO: 5 |
| FR3 | SEQ ID NO: 11 |
| FR4 | SEQ ID NO: 12 |

Further, the VHH domain comprises an amino acid sequence set forth in SEQ ID NO: 8 with or without 1-11 (1, 2, 3, 4, 5, 6, 7, 8, 9, 10 or 11) amino acid mutations. Further, the amino acid mutation is at a position selected from the group consisting of positions 1, 5, 49, 58, 65, 74, 82B, 83, 84, 89, 108, and a combination thereof. Further, the amino acid mutation is selected from the group consisting of Q1E, Q5L, A49S, N58Y, D65G, D74S, V(82B)S, K83R, P84A, M89V, Q108L and a combination thereof. Further preferably, the amino acid mutation is selected from the following mutation combinations:

| | |
|---|---|
| Mutation combination 1 | Q1E+Q5L+A49S+N58Y+D65G+D74S+V(82B)S+K83R+P84A+M89V+Q108L |
| Mutation combination 2 | Q1E+Q5L+N58Y+D65G+D74S+V(82B)S+K83R+P84A+M89V+Q108L |

Further preferably, the VHH domain comprises an amino acid sequence set forth in SEQ ID NO: 8, SEQ ID NO: 13 or SEQ ID NO: 14.

The present disclosure further relates to use of the VHH domain in the construction of a recombinant protein.

The present disclosure further relates to a recombinant protein comprising the VHH domain targeting VEGF.

Further, the recombinant protein is selected from the group consisting of a bispecific antibody, a multispecific antibody, and an antibody-drug conjugate.

The present disclosure further relates to a polynucleotide encoding any of the aforementioned bispecific antibody, multispecific antibody, recombinant protein or VHH domain.

The present disclosure further relates to an expression vector comprising any of the aforementioned polynucleotide.

The present disclosure further relates to a host cell comprising any one of the aforementioned expression vector or integrated with any one of the aforementioned polynucleotide.

The present disclosure further relates to a pharmaceutical composition, comprising any of the aforementioned VHH domain, bispecific antibody, multispecific antibody or recombinant protein, and a pharmaceutically acceptable carrier.

The present disclosure further relates to use of any of the aforementioned bispecific antibody, multispecific antibody, recombinant protein or VHH domain in the manufacture of a medicament for treating cancer.

The present disclosure further relates to a method for treating cancer, comprising administering to a subject in need thereof an effective amount of any one of the aforementioned VHH domain, bispecific antibody, multispecific antibody or recombinant protein.

Further, in any of the aforementioned use or method, the cancer is colorectal cancer.

The beneficial effects of the present disclosure are as follows. The VHH domain targeting VEGF, which has been screened and optimized by the present disclosure, exhibits a stable structure, high thermal stability, and high binding activity with both human VEGF-A and mouse VEGF-A. This enables simultaneous verification in animal models, thereby improving the success rate of drug development. Furthermore, it has the characteristics of high protein expression and being conducive to purification. The bispecific antibody developed based on the VHH domain of the present disclosure has a unique bispecific antibody structure, a high degree of humanization, does not contain artificially added redundant sequences, and has the potential to be locally enriched in tumors with high VEGF expression, thereby exerting a more targeted tumor inhibition effect, achieving a more effective tumor inhibition effect, and solving the problem of insufficient efficacy of PD-1/PD-L1 inhibitors.

### BRIEF DESCRIPTION OF DRAWINGS

FIG. 1 shows the binding of antibodies to VEGF after multiple rounds of combined mutations.
FIG. 2 shows the schematic diagram of the structure of a bispecific antibody.
FIG. 3 shows the level of interleukin 2 (IL-2) released by the tested antibodies in the MLR reaction.
FIG. 4 shows the level of interferon-γ (IFN-γ) released by the tested antibodies in the MLR reaction.
FIG. 5 shows the relative inhibition rate of the HUVEC cell proliferation by the tested antibodies.
FIG. 6 shows the inhibition of the migration of HUVEC cells by the tested antibodies.
FIG. 7 shows the amount of antibody remaining after binding to VEGF magnetic beads, which was quantified by the ELISA method.
FIG. 8 shows the tumor volume in tumor-bearing mouse models treated with the tested antibodies.

### DETAILED DESCRIPTION

### [Definitions]

Unless otherwise specifically stated, the practice of the present disclosure will be carried out using conventional methods of virology, immunology, microbiology, molecular biology and recombinant DNA technology within the technical scope of the art or as generally understood by those of ordinary skill in the art to which the present disclosure belongs. Many of these are described below for illustrative purposes, and such techniques are fully described in the literatures.

As used herein, the term "antibody" refers to an antibody in its substantially complete form, as opposed to an antibody fragment. Specifically, the "antibody" involved herein is preferably an antibody in its complete form, which has a symmetrical structure with four polypeptide chains, two heavy chains (H chains) and two light chains (L chains), and the chains are linked by disulfide bonds and non-covalent bonds to form a monomer molecule composed of four polypeptide chains.

As used herein, the term "antigen-binding fragment" comprises a moiety of an intact antibody, preferably the antigen-binding region and/or variable region of an intact antibody. Examples of antibody fragments include Fab, Fab', F(ab')₂ and Fv fragments, etc.

As used herein, the term "humanized antibody or antigen-binding fragment" is a human immunoglobulin (receptor antibody), wherein the residues from the HVR of the receptor are replaced by residues from non-human species (donor antibody) with desired specificity, affinity, and/or ability, such as mice, rats, rabbits or non-human primates. In some cases, the framework ("FR") residues of human immunoglobulin are replaced by corresponding non-human residues. In addition, humanized antibodies or antigen-binding fragments may include residues that are not present in the receptor antibody or donor antibody. These modifications can be made to further improve antibody performance, such as binding affinity. Generally speaking, humanized antibodies or antigen-binding fragments will include at least one, and generally substantially all of two variable domains, wherein all or substantially all of the hypervariable loops correspond to those of non-human immunoglobulin sequences, and all or substantially all of the FR regions are those of human immunoglobulin sequences, but the FR regions may include one or more separate FR residues that improve antibody performance, such as binding affinity, isomerization, immunogenicity, etc. Humanized antibodies optionally will further comprise at least a moiety of an immunoglobulin constant region (Fc), typically that of a human immunoglobulin. For additional details, see, e.g., Jones et al., Nature 321: 522-525 (1986); Riechmann et al., Nature 332: 323-329 (1988); and Presta, Curr. Op. Struct. Biol. 2: 593-596 (1992). See also, for example, Vaswani and Hamilton, Ann. Allergy, Asthma & Immunol. 1: 105-115 (1998); Harris, Biochem. Soc. Transactions 23: 1035-1038 (1995); Hurle and Gross, Curr. Op. Biotech. 5: 428-433 (1994); and U.S. Pat. Nos. 6,982,321 and 7,087,409.

As used herein, the term "human antibody" is an antibody comprising an amino acid sequence corresponding to an antibody produced by a human and/or prepared using any of the techniques for preparing human antibodies. This definition of a human antibody specifically excludes humanized antibodies comprising non-human antigen binding residues. Human antibodies can be prepared using a variety of techniques known in the art, including phage display libraries. Hoogenboom and Winter, J. Mol. Biol., 227: 381 (1991); Marks et al., J. Mol. Biol., 222: 581 (1991). Also useful for preparing human monoclonal antibodies are the methods described in Cole et al., Monoclonal Antibodies and Cancer Therapy, Alan R. Liss, p. 77 (1985); Boerner et al., J. Immunol., 147 (1): 86-95 (1991). See also van Dijk and van de Winkel, Curr. Opin. Pharmacol., 5: 368-74 (2001). Human antibodies can be prepared by administering an antigen to a transgenic animal that has been modified to produce such antibodies in response to antigenic challenge, but whose endogenous loci have been disabled, such as immunized heterologous mice (see, e.g., U.S. Pat. Nos. 6,075,181 and 6,150,584 regarding XENOMOUSE^{™} technology). See also, e.g., Li et al., Proc. Natl. Acad. Sci. USA, 103: 3557-3562 (2006), regarding human antibodies produced via human B cell hybridoma technology.

As used herein, the term "CDR" (i.e., complementarity determining region) is used to refer to a hypervariable region, as defined by the Kabat system. See Kabat et al., Sequences of Proteins of Immunological Interest, 5th edition, Public Health Service, National Institutes of Health, Bethesda, Md. (1991). There are other definitions for CDR, such as IMGT, Chothia, AbM, Contact, etc.

Unless otherwise indicated, the numbering of immunoglobulin residues referred to herein is that of the Kabat index.

As used herein, the term "Fc domain" generally includes the hinge region, the heavy chain constant region CH2, and the heavy chain constant region CH3 (i.e., Hinge-CH2-CH3). The Fc domain forms a dimer typically via a disulfide bond. At present, the most commonly used fusion partner is the Fc fragment of immunoglobulin IgG. The antibody Fc is a part of the antibody constant region (including the hinge region-CH2-CH3, but not the CH1 part of the antibody constant region). After fusion with the Fc fragment, the fusion molecule has increased stability and prolonged half-life *in vivo* due to the increased molecular weight and the recycling mechanism mediated by FcRn. Meanwhile, the Fc fragment can be used to mediate various biological functions such as ADCC and CDC. However, this type of fusion protein does not have the variable region of the antibody, and its pharmacology and efficacy primarily depend on the functional molecule fused with Fc. There are 4 subtypes of human immunoglobulin G, and the biological activities of different immunoglobulin G subtypes vary. The most widely used is the IgG1 antibody currently. In recent years, with the expansion of new indications and the emergence of antibody drugs with new mechanisms of action, IgG2 and IgG4 subtypes with lower cytotoxicity have been paid attention to. In the present disclosure, the "Fc domain" is preferably the Fc domain of an IgG1 antibody or the Fc domain of an IgG4 antibody.

As used herein, the term "specificity" refers to an antigen binding protein or antibody that selectively recognizes a specific epitope of an antigen. For example, natural antibodies are monospecific. As used herein, the term "bispecific" or "multispecific" means that an antigen binding protein or antibody has two or more antigen binding sites, at least two of which bind to different antigens or different epitopes of the same antigen.

As used herein, the terms "bivalent", "trivalent" and "tetravalent" are associated with "valency", which indicates the specified number of binding sites present in an antigen binding protein or antibody molecule. Thus, the terms "bivalent", "trivalent" and "tetravalent" indicate the presence of two binding sites, three binding sites and four binding sites, respectively, in an antigen binding protein or antibody molecule.

"VHH domain" (variable domain of heavy chain of heavy-chain antibody), also known as VHH or VHH antibody fragment, originally derived from the antigen-binding immunoglobulin variable domain of "heavy-chain antibody" (hcAb, i.e. "antibody devoid of light chain") >C. Hamers-Casterman, T. Atarhouch, et al. Naturally occurring antibodies devoid of light chains. Nature, June 3, 1993, Vol. 363, pp. 446-448). The term "VHH domain" is used to distinguish this type of variable domain from the heavy chain variable domain (which is referred to as "VH domain" herein) present in conventional antibodies and the light chain variable domain (which is referred to as "VL domain" herein) present in conventional antibodies composed of two light chains and two heavy chains (hereinafter referred to as conventional antibodies). The VHH domain specifically binds to the epitope without the need for other antigen-binding domains (this is in contrast to the VH or VL domain in conventional antibodies, in which the VL domain and the VH domain are combined to recognize the epitope). The VHH domain is an antigen recognition unit formed by a single immunoglobulin domain.

Herein, the terms "single domain antibody (sdAb)", "VHH domain", "VHH", "VHH antibody fragment", "VHH antibody", Nanobody and "Nanobody domain" are used interchangeably.

As used herein, the term "identity" is the percentage of amino acid residues in a candidate sequence that are identical to the amino acid residues in a particular peptide or polypeptide sequence, after aligning the sequences and introducing gaps (if necessary) to achieve the maximum sequence identity percentage, but without considering any conservative substitutions as part of the sequence identity. Alignment for the purpose of determining amino acid sequence identity percentage can be achieved in various ways within the skill of the art, for example, using publicly available computer software such as BLAST, BLAST-2, ALIGN or MEGALIGN^{™} (DNASTAR) software. Those skilled in the art can determine appropriate parameters for measuring alignment, including any algorithm required for achieving maximum alignment over the full length of the compared sequences.

As used herein, the term "treatment" refers to a clinical intervention designed to change the natural course of an individual or cell treated in a clinical pathology process. The desired therapeutic effect includes reducing the rate of disease progression, improving or alleviating the disease state, and ameliorating or improving prognosis. For example, one or more symptoms associated with a disease or condition (such as cancer, inflammation, or autoimmune disease) treated are alleviated or eliminated.

As used herein, "effective amount" refers to the amount of an agent or drug that effectively treats a disease or condition in a subject. In the case of cancer, the effective amount of the anti-VEGF single-domain antibody binding fragment, bispecific antibody, multi-specific antigen-binding construct, pharmaceutical composition, and immunoconjugate provided by the present disclosure can decrease the number of cancer cells, reduce tumor size, inhibit (i.e., slow to a certain extent and preferably prevent) cancer cell infiltration into peripheral organs, suppress (i.e., slow to a certain extent and preferably prevent) tumor cancer cell metastasis, inhibit tumor growth to a certain extent; and/or alleviate one or more symptoms associated with cancer to a certain extent. As understood in the clinical environment, an effective amount of a drug, compound, or pharmaceutical composition may or may not be achieved in combination with another drug, compound, or pharmaceutical composition. Therefore, an "effective amount" can be considered in the context of administering one or more therapeutic agents, and if combined with one or more other agents, it can be considered that a single agent given in an effective amount can achieve the desired result.

As used herein, a "subject" is preferably a mammal, including but not limited to humans, cattle, horses, felines, canines, rodents, or primates. In some embodiments, the subject is a human.

### [Specific Embodiments]

The embodiments of the present disclosure will be described in detail below in conjunction with examples, but those skilled in the art will appreciate that the following examples are only used to illustrate the present disclosure and should not be construed as limiting the scope of the present disclosure.

### Example 1 Screening and development of VEGF-targeted VHHs

500 µg of human and mouse VEGF-A proteins were mixed with an equal volume of an adjuvant to prepare an immunogen, with which a 2-year-old female alpaca was immunized 4 times, with an interval of 2 weeks each time. After immunization was completed, hyperimmune serum was collected, peripheral blood mononuclear cells were separated, and RNA was extracted. The variable region gene of the nanobody was amplified by RT-PCR. The VEGF-A immune antibody library was obtained by enzyme digestion, ligation, and electroporation.

For the prepared VEGF-A immune antibody library, two rounds of panning were performed using immunotube solid phase panning and magnetic bead liquid phase screening, and single clones were picked for ELISA detection. The positive clones were sequenced, and 81 different antibody genes were finally obtained. The VHH-Fc DNA fragments were constructed for all 81 strains obtained. After sequencing, 293T cells were transfected for eukaryotic expression and the expression supernatant was collected for ELISA specific detection. After testing, it was found that strain 6 (#6) was the only antibody with excellent binding ability to both human and mouse VEGF-A (see Tables 7 and 8).

**Table 7 Binding ability of #6 to human VEGF-A**

| | |
|---|---|
| VHH Number | EC50 (ng/mL) |
| #6 | 9.331 |
| Bevacizumab | 36.56 |

**Table 8 Binding ability of #6 to mouse VEGF-A**

| | |
|---|---|
| VHH Number | EC50 (ng/mL) |
| #6 | 6.46 |
| Bevacizumab | - |

Strain 6 (#6) was sequenced, and its amino acid sequence is as follows:

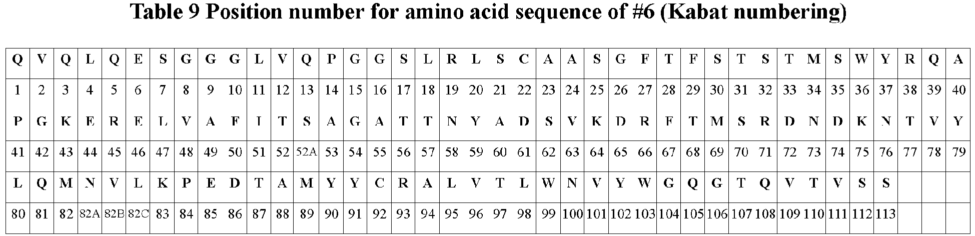

SEQ ID No: 8 (CDRs 1-3 (Kabat numbering) are bold and underlined)

QVQLQESGGGLVQPGGSLRLSCAASGFTFSTSTMSWYRQAPGKERELVAFITSAGATTNYADSVKDR

FTMSRDNDKNTVYLQMNVLKPEDTAMYYCRALVTLWNVYWGQGTQVTVSS

### Example 2 Humanization and stability optimization of the original VEGF VHH sequence

In order to further improve the thermal stability and humanization of strain 6 VHH, combined with protein structure simulations based on structural biology, five rounds of sequence optimization and modification were performed to obtain two VHH sequences with good thermal stability and high humanization.
#6 (SEQ ID NO: 8): 
QVQLQESGGGLVQPGGSLRLSCAASGFTFSTSTMSWYRQAPGKERELVAFITSAGATTN
YADSVKDRFTMSRDNDKNTVYLQMNVLKPEDTAMYYCRALVTLWNVYWGQGTQVT
VSS
#48 (SEQ ID NO: 13): 
EVQLLESGGGLVQPGGSLRLSCAASGFTFSTSTMSWYRQAPGKERELVSFITSAGATTYY
ADSVKGRFTMSRDNSKNTVYLQMNSLRAEDTAVYYCRALVTLWNVYWGQGTLVTVS
S
#49 (SEQ ID NO: 14):

EVQLLESGGGLVQPGGSLRLSCAASGFTFSTSTMSWYRQAPGKERELVAFITSAGATTY
YADSVKGRFTMSRDNSKNTVYLQMNSLRAEDTAVYYCRALVTLWNVYWGQGTLVTV
SS

**Table 10 Amino acid mutation sites after multiple rounds of sequence optimization (using the Kabat system position in Table 9)**

| No. | Mutation site relative to #6 VHH sequence | Identity to #6 VHH sequence |
|---|---|---|
| #48 | Q1E+Q5L+A49S+N58Y+D65G+D74S+V(82B)S+K83R +P84A+M89V+Q108L | 106/117(91%) |
| #49 | Q1E+Q5L+N58Y+D65G+D74S+V(82B)S+K83R+P84A + M89V+Q108L | 107/117(91%) |

For the #48 sequence, Q1E+Q5L is located in the FR1 region, A49S is located in the FR2 region, N58Y+D65G is located in the CDR2 region, D74S+V(82B)S+K83R+P84A+M89V is located in the FR3 region, and Q108L is located in the FR4 region.

For the #49 sequence, Q1E+Q5L is located in the FR1 region, N58Y+D65G is located in the CDR2 region, D74S+V(82B)S+K83R+P84A+M89V is located in the FR3 region, and Q108L is located in the FR4 region.

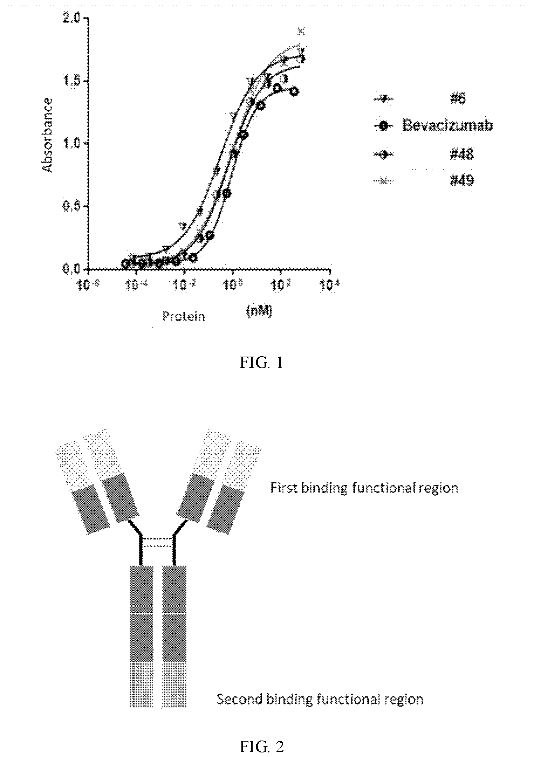

The results of VEGF binding ability and thermal stability assay show that #48 and #49 both exhibited good VEGF binding ability comparable to #6 (see FIG. 1). The thermal stability results show that the thermal stability of the combined mutation sequences increased by nearly 10°C, with Tm values of 64.1°C and 64.7°C, respectively.

### Example 3 Screening of molecular structures of candidate bispecific antibody and construction of control substances

Based on #48 VHH and the antigen binding region fragments of the anti-PD-1 antibody nivolumab (Opdivo) or pembrolizumab (Keytruda), various bispecific antibody structures were evaluated and screened. After comprehensive evaluation of activity, expression, stability, etc., the bispecific antibody structure shown in FIG. 2 was selected. The #10 and #16 bispecific antibody molecules were constructed for further evaluation. The valences were both divalent + divalent (2+2), and Fc was a functionally silent IgG1 mutation Fc (AEASS). The amino acid sequences of the constructed molecules are shown in Table 11.

**Table 11 Amino acid sequence of the constructed molecule**

| |
|---|
| #10 |
| Heavy chain (SEQ ID NO: 20): |
| QVQLVESGGG VVQPGRSLRL DCKASGITFS NSGMHWVRQA PGKGLEWVAV IWYDGSKRYY 60 ADSVKGRFTI SRDNSKNTLF LQMNSLRAED TAVYYCATND DYWGQGTLVT VSSASTKGPS 120 VFPLAPSSKS TSGGTAALGC LVKDYFPEPV TVSWNSGALT SGVHTFPAVL QSSGLYSLSS 180 VVTVPSSSLG TQTYICNVNH KPSNTKVDKK VEPKSCDKTH TCPPCPAPEA EGAPSVFLFP 240 PKPKDTLMIS RTPEVTCVVV DVSHEDPEVK FNWYVDGVEV HNAKTKPREE QYNSTYRVVS 300 VLTVLHQDWL NGKEYKCKVS NKALPSSIEK TISKAKGQPR EPQVYTLPPS REEMTKNQVS 360 LTCLVKGFYP SDIAVEWESN GQPENNYKTT PPVLDSDGSF FLYSKLTVDK SRWQQGNVFS 420 CSVMHEALHN HYTQKSLSLS PGEVQLLESG GGLVQPGGSL RLSCAASGFT FSTSTMSWYR 480 QAPGKERELV SFITSAGATT YYADSVKGRF TMSRDNSKNT VYLQMNSLRA EDTAVYYCRA 540 LVTLWNVYWG QGTLVTVSS 559 |
| Light chain (SEQ ID NO: 21): |
| EIVLTQSPAT LSLSPGERAT LSCRASQSVS SYLAWYQQKP GQAPRLLIYD ASNRATGIPA 60 RFSGSGSGTD FTLTISSLEP EDFAVYYCQQ SSNWPRTFGQ GTKVEIKRTV AAPSVFIFPP 120 SDEQLKSGTA SVVCLLNNFY PREAKVQWKV DNALQSGNSQ ESVTEQDSKD STYSLSSTLT 180 LSKADYEKHK VYACEVTHQG LSSPVTKSFN RGEC 214 |
| #16 |
| Heavy chain (SEQ ID NO: 22): |
| QVQLVQSGVE VKKPGASVKV SCKASGYTFT NYYMYWVRQA PGQGLEWMGG INPSNGGTNF 60 NEKFKNRVTL TTDSSTTTAY MELKSLQFDD TAVYYCARRD YRFDMGFDYW GQGTTVTVSS 120 ASTKGPSVFP LAPSSKSTSG GTAALGCLVK DYFPEPVTVS WNSGALTSGV HTFPAVLQSS 180 GLYSLSSVVT VPSSSLGTQT YICNVNHKPS NTKVDKKVEP KSCDKTHTCP PCPAPEAEGA 240 PSVFLFPPKP KDTLMISRTP EVTCVVVDVS HEDPEVKFNW YVDGVEVHNA KTKPREEQYN 300 STYRVVSVLT VLHQDWLNGK EYKCKVSNKA LPSSIEKTIS KAKGQPREPQ VYTLPPSREE 360 |
| MTKNQVSLTC LVKGFYPSDI AVEWESNGQP ENNYKTTPPV LDSDGSFFLY SKLTVDKSRW 420 QQGNVFSCSV MHEALHNHYT QKSLSLSPGE VQLLESGGGL VQPGGSLRLS CAASGFTFST 480 STMSWYRQAP GKERELVSFI TSAGATTYYA DSVKGRFTMS RDNSKNTVYL QMNSLRAEDT 540 AVYYCRALVT LWNVYWGQGT LVTVSS 566 |
| Light chain (SEQ ID NO: 23): |
| EIVLTQSPAT LSLSPGERAT LSCRASKGVS TSGYSYLHWY QQKPGQAPRL LIYLASYLES 60 GVPARFSGSG SGTDFTLTIS SLEPEDFAVY YCQHSRDLPL TFGGGTKVEI KRTVAAPSVF 120 IFPPSDEQLK SGTASVVCLL NNFYPREAKV QWKVDNALQS GNSQESVTEQ DSKDSTYSLS 180 STLTLSKADY EKHKVYACEV THQGLSSPVT KSFNRGEC 218 |

A bispecific antibody AK112 analog targeting PD-1 and VEGF, in-house prepared based on the heavy chain amino acid sequence of SEQ ID NO: 25 and the light chain amino acid sequence of SEQ ID NO: 26, was used as a control.
SEQ ID NO: 25: 
EVQLVESGGGLVQPGGSLRLSCAASGYTFTNYGMNWVRQAPGKGLEWVGWINTYTGE
PTYAADFKRRFTFSLDTSKSTAYLQMNSLRAEDTAVYYCAKYPHYYGSSHWYFDVWGQ
GTLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCPA
PEAAGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGGGGGSGGGGSGGGGSGGGGSE
VQLVESGGGLVQPGGSLRLSCAASGFAFSSYDMSWVRQAPGKGLDWVATISGGGRYTYY
PDSVKGRFTISRDNSKNNLYLQMNSLRAEDTALYYCANRYGEAWFAYWGQGTLVTVSSG
GGGSGGGGSGGGGSGGGGSDIQMTQSPSSMSASVGDRVTFTCRASQDINTYLSWFQQKP
GKSPKTLIYRANRLVSGVPSRFSGSGSGQDYTLTISSLQPEDMATYYCLQYDEFPLTFGAG
TKLELK
SEQ ID NO: 26:

DIQMTQSPSSLSASVGDRVTITCSASQDISNYLNWYQQKPGKAPKVLIYFTSSLHSGVPSR
FSGSGSGTDFTLTISSLQPEDFATYYCQQYSTVPWTFGQGTKVEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC

### Example 4 Binding to human PD-1 and human VEGF

The binding of the antibody to human PD-1 was detected by ELISA. 1 µg/mL human PD-1 (ACRO, His tag) was coated on an ELISA plate overnight and blocked. Subsequently, the plate was added with gradient dilutions of the bispecific antibody and the control antibody, incubated and washed. Then, the plate was added with HRP-labeled anti-human IgG Fc secondary antibody, incubated and washed. After TMB solution was added for color development, the OD450 absorbance value was read on a microplate reader (see Table 12). The binding of the antibody to human VEGF was detected by ELISA. 50 ng/mL human VEGF (ACRO, His tag) was coated on an ELISA plate overnight and blocked. Subsequently, the plate was added with gradient dilutions of the sample to be tested and the control antibody, incubated and washed. Then, the plated was added with HRP-labeled anti-human IgG Fc secondary antibody, incubated and washed. After TMB solution was added for color development, the OD450 absorbance value was read on a microplate reader (see Table 13). From the results of the ELISA detection, it can be seen that the ability of #10 and #16 binding to human PD-1 was comparable to that of Opdivo and Keytruda, but with a higher Top signal and a stronger binding ability than that of the control AK112 analog. The binding pattern of #10 and #16 to VEGF was very unique. Compared with the binding ability of #48 to VEGF, the ability of #10 and #16 binding to VEGF was slightly reduced, and the Top absorbance value was about half of that of #48, indicating that the optimization based on structural biology had changed the state of #10 and #16 binding to VEGF, thereby achieving more drug enrichment. Bevacizumab antibody fragment was used as the anti-VEGF part of the AK112 analog, which exhibited similar characteristics to Bevacizumab in terms of VEGF binding. (The control substances were all obtained by in-house expression and purification)

**Table 12 Binding of the tested antibodies to human PD-1**

| Protein molecules | Molecular weight (kD) | EC50 (nM) | TOP OD |
|---|---|---|---|
| #10 | 169.3 | 0.114 | 2.64 |
| #16 | 171.9 | 0.114 | 2.579 |
| Opdivo | 143 | 0.1 | 2.271 |
| Keytruda | 146 | 0.102 | 2.324 |
| AK112 analog | 201.5 | 0.528 | 2.19 |
| Bevacizumab | 146.29 | - | 0.151 |

**Table 13 Binding of the tested antibodies to human VEGF**

| Protein molecules | Molecular weight (kD) | EC50 (nM) | TOP OD |
|---|---|---|---|
| #10 | 169.3 | 0.366 | 1.752 |
| #16 | 171.9 | 0.579 | 1.745 |
| #48 | 76.46 | 0.608 | 3.091 |
| Opdivo | 143 | - | 0.048 |
| AK112 analog | 201.5 | 0.64 | 2.683 |
| Bevacizumab | 146.29 | 0.374 | 3.157 |

### Example 5 Dual Binding Ability to Human VEGF and Human PD-1

To further verify the ability of #10, #16, and AK112 analog dually binding to human VEGF and human PD-1, a 96-well ELISA plate was coated with 50 ng/mL of human VEGF (Sino Biological, no label) overnight, washed and blocked for 1 hour. Subsequently, the plate was added with gradient dilutions of the test antibody, incubated for 2 hours, then added with 1 µg/mL of human PD-1 (ACRO, His tag), incubated for 2 hours, washed, and incubated with HRP-labeled anti-His antibody for 1 hour. After TMB solution was added for color development, the OD450 nm absorbance value was read on a microplate reader. The results are shown in Table 14. A single targeting antibody failed to bind to two targets simultaneously. #10, #16, and AK112 analog all had the ability to dually bind to human VEGF and human PD-1, with EC50 values of 0.279 nM, 0.326 nM, and 0.254 nM, respectively.

The order of the binding to VEGF and PD-1 was changed for dual ELISA detection. A 96-well ELISA plate was coated with 1 µg/mL human PD-1 (ACRO, His tag) overnight, washed and blocked for 1 hour. Subsequently, the plate was added with gradient dilutions of specific antibodies, incubated for 2 hours, then added with 50 ng/mL of biotin-labeled human VEGF (ACRO, biotinylated protein), incubated for 2 hours, washed, and incubated with HRP-labeled streptavidin for 1 hour. TMB solution was added for color development, and the OD450 nm absorbance value was read on a microplate reader. The results are shown in Table 14. A single targeting antibody failed to bind to two targets simultaneously. #10, #16 and AK112 analog all had the ability to dually bind to human PD-1 and human VEGF, with EC50 values of 0.114 nM, 0.117 nM and 0.634 nM, respectively. Based on the comparison of EC50 values, the ability of #10 and #16 dually binding to human PD-1 and human VEGF was 5-6 times stronger than that of the competitor AK112 analog.

**Table 14 Dual binding of the tested antibodies to human PD-1 and human VEGF**

| Protein molecules | Molecular weight (kD) | EC50 (nM) | TOP OD |
|---|---|---|---|
| #10 | 169.3 | 0.114 | 3.571 |
| #16 | 171.9 | 0.117 | 3.649 |
| #48 | 76.46 | - | 0.061 |
| Opdivo | 143 | - | 0.048 |
| AK112 analog | 201.5 | 0.634 | 3.415 |
| Bevacizumab | 146.29 | - | 0.067 |

### Example 6 Binding of antigens of different species

The binding of candidate molecules and competitor to antigens of different species was detected by ELISA. The monkey VEGF sequence was completely identical to the human VEGF sequence, and the candidate antibody had the same ability to bind to monkey VEGF as that of human VEGF. The binding of antibodies to monkey PD-1 was detected by ELISA. An ELISA plate was coated with 1 µg/mL monkey PD-1 (ACRO, His tag) overnight and blocked. Subsequently, the plate was added with gradient dilutions of the sample to be tested and the control antibody, incubated and washed. Then, the plate was added with HRP-labeled anti-human IgG Fc secondary antibody, incubated and washed. After TMB solution was added for color development, the OD450 absorbance value was read on a microplate reader (see Table 15). Consistent with the results of binding to human PD-1, the ability of #10 and #16 binding to monkey PD-1 was comparable to that of Opdivo and Keytruda, and stronger than the competitor AK112 analog.

**Table 15 Binding of the tested antibodies to monkey PD-1**

| Protein molecules | Molecular weight (kD) | EC50 (nM) | TOP OD |
|---|---|---|---|
| #10 | 169.3 | 0.229 | 3.359 |
| #16 | 171.9 | 0.171 | 3.376 |
| Opdivo | 143 | 0.153 | 2.987 |
| Keytruda | 146 | 0.187 | 3.078 |
| AK112 analog | 201.5 | 1.704 | 2.561 |
| Bevacizumab | 146.29 | - | 0.127 |

### Example 7 Ability to block the binding of PD-1 and PD-L1

The ability of the tested antibodies to block the binding of PD-1 and PD-L1 was further detected. An ELISA plate was coated with 1 µg/mL of human PD-1 (ACRO, Fc tag) overnight, washed and blocked. Subsequently, the plate was added with a mixture of gradient dilutions of antibodies and human PD-L1 (Sino Biological, His tag), and incubated for 2 hours. Then the plate was added with HRP-labeled anti-His antibody, and incubated for 1 hour. After TMB solution was added for color development, the OD450 absorbance value was read on a microplate reader (see Table 16). As shown in the results, except for #48, which only bound to VEGF, the remaining antibodies can block the binding of PD-1 and PD-L1, and the blocking activity thereof was comparable.

**Table 16 Detection of the ability of the tested antibodies to block the binding of human PD-1 to human PD-L1**

| Protein molecules | IC50 (nM) |
|---|---|
| #10 | 2.419 |
| #16 | 2.529 |
| #48 | - |
| Opdivo | 1.947 |
| Keytruda | 2.13 |
| AK112 analog | 2.898 |

### Example 8 Ability to block the binding of VEGF to VEGFR1 and VEGFR2

The ability of the tested antibodies to block the binding of VEGF to VEGFR1 and VEGFR2 was detected. ELISA plates were respectively coated with 2 µg/mL human VEGFR1 and 5 µg/mL human VEGFR2 (in-house constructed and expressed, Fc tag) overnight, washed and blocked. Subsequently, the plates were added with a mixture of gradient dilutions of antibodies and human VEGF (ACRO, biotinylated protein), incubated for 2 hours, and washed. Then the plates were added with HRP-labeled streptavidin and incubated for 1 hour. After TMB solution was added for color development, the OD450 nm absorbance value was read on a microplate reader. The results show that #10 and #16 had stronger ability to block the binding of VEGF and VEGFR1, with IC50 values of 0.491 nM and 0.507 nM, respectively, which were better than the blocking ability of #48, exhibiting the advantages of the changes in the spatial structure of the constructed bispecific antibodies. The blocking ability of the competitor AK112 analog was comparable to that of Bevacizumab, with IC50 values of 4.610 nM and 5.647 nM, respectively. In addition, the results show that #10 and #16 had stronger ability to block the binding of VEGF and VEGFR2, with IC50 values of 3.643 nM and 3.249 nM, respectively, which were better than the blocking ability of #48 with an IC50 value of 5.841 nM, exhibiting the advantages of the change in the spatial structure of bispecific antibodies. The blocking ability of the competitor AK112 analog was comparable to that of Bevacizumab, with IC50 values of 6.481 nM and 6.637 nM, respectively.

### Example 9 Response of T cells after blocking PD-1 verified by mixed lymphocyte reaction (MLR) method

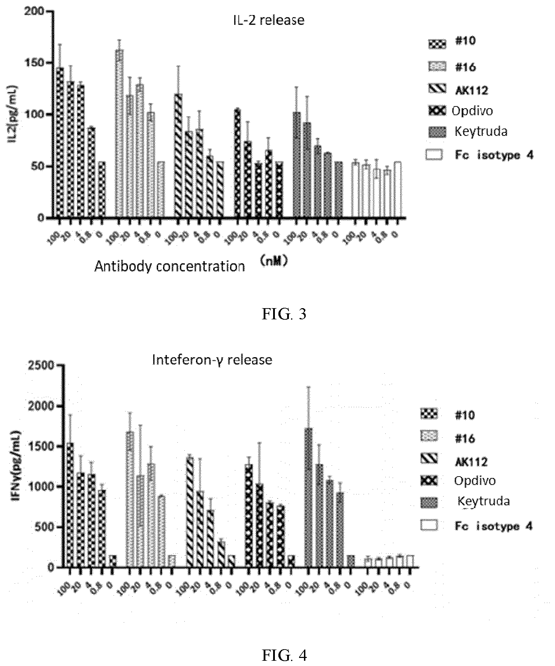

The functions of the candidate molecules and competitor AK112 analog were further compared at the cellular level. The response of T cells after blocking PD-1 was verified by the mixed lymphocyte reaction (MLR) method. 2×10⁴ DCs from one donor and 2×10⁵ CD4⁺ T cells from another donor were mixed in equal volumes, added with gradient dilutions of the test antibodies and cultured for 72 hours. The cell culture supernatant was collected, and the contents of IL-2 (FIG. 3) and IFNγ (FIG. 4) released in the cell supernatant was measured by the ELISA quantitative kit. The results show that the contents of IL-2 and IFNγ released by CD4⁺ T cells in the MLR reaction promoted by #10 and #16 were higher than that of the competitor AK112 analog, indicating that #10 and #16 had a stronger effect on T cells.

### Example 10 Ability of test antibodies to inhibit proliferation of VEGF-stimulated human umbilical vein endothelial cell (HUVEC) verified by HUVEC proliferation inhibition assay

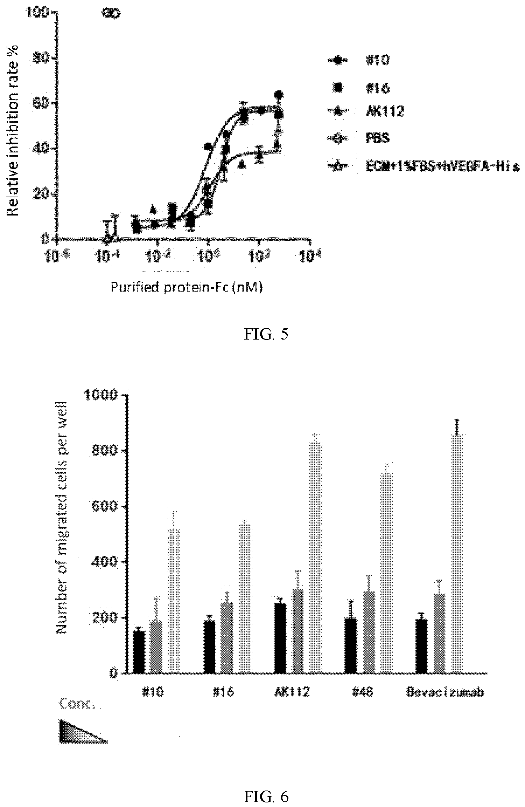

5×10³ HUVEC cells/well were added to a 96-well cell plate in a medium containing 1% FBS and cultured overnight in a 37°C, 5% carbon dioxide incubator. The next day, the medium was removed, and 70 ng/mL VEGF and gradiently diluted antibodies to be tested and competitor AK112 analog were added. The reaction plate was placed in a 37°C, 5% carbon dioxide incubator for 4 days. After the plate was added with CTG reagent, the relative fluorescence signal value was read on a microplate reader. The fluorescence value of the ECM medium containing 1% FBS and the VEGF-stimulated cell group (without antibody) was defined as an inhibition rate of 0, and the fluorescence value of the PBS group (without ECM medium containing 1% FBS, and without VEGF stimulation and antibody) was defined as 100% inhibition rate. The relative inhibition rate was fitted into a curve. As shown in FIG. 5, the maximum inhibition rate of #10 and #16 was about 60%, while the maximum inhibition rate of AK112 analog was about 40%. The inhibition rates of #10 and #16 were higher than that of the competitor AK112 analog.

### Example 11 Ability to inhibit HUVEC cell migration

The ability of the test antibodies to inhibit HUVEC cell migration was detected by a Transwell chamber with a well size of 8 µm. The Transwell consists of an upper chamber and a lower chamber. 5×10³ HUVEC cells/well were added to the upper chamber and cultured in a 37°C, 5% carbon dioxide incubator for 30 minutes. 200 ng/mL VEGF and different concentrations of the test antibodies or the competitor AK112 analog were added to the lower chamber, three replicate wells for each group. The cells was cultured in a 37°C, 5% carbon dioxide incubator for 24 hours, then fixed with 4% paraformaldehyde and stained with crystal violet. The number of cells that migrated to the back of the upper chamber was counted after taking pictures. The fewer the number of cells, the higher the inhibition rate. The results in FIG. 6 show that the inhibition rates of #10 and #16 were higher than that of the competitor AK112 analog.

### Example 12: Enhanced antibody enrichment properties

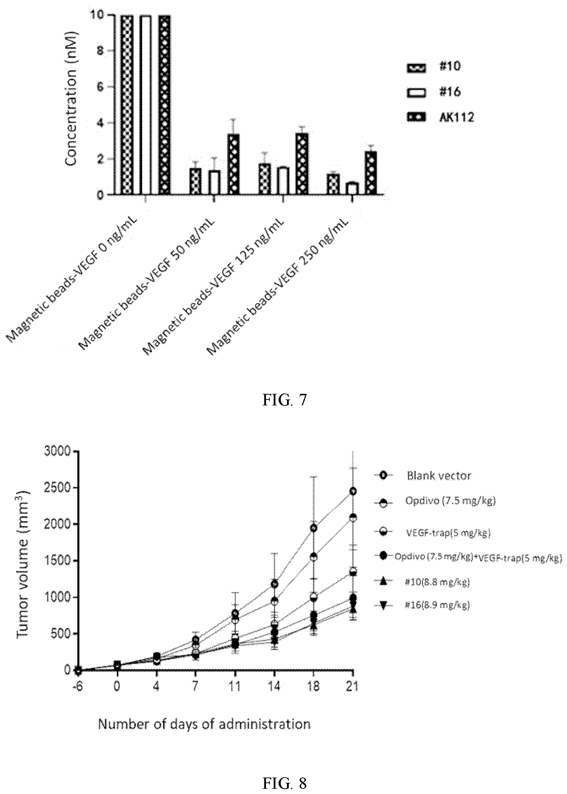

Structural biology simulations show that the conformation of #10 may change the mode of binding of anti-VEGF nanobody to VEGF, and antibodies can be enriched through dimeric VEGF proteins. In order to verify the characteristics of #10 in increasing antibody enrichment, magnetic beads coupled to human VEGF protein (ACRO) were diluted to 250 ng/mL, 125 ng/mL and 50 ng/mL with PBS, added with 10 nM of #10, #16 and competitor AK112 analog, incubated for 1 hour, and then placed on a magnet for two minutes. The supernatant was collected for ELISA protein quantification. The results are shown in FIG. 7. The amount of remaining antibodies of #10 and #16 was less than that of competitor AK112 analog, indicating that the magnetic beads containing VEGF enriched more bispecific antibodies. This quantitative ELISA experiment was to verify the degree of cross-linking between antibodies and VEGF. The antibodies were first cross-linked with VEGF magnetic beads, and the remaining amount of antibodies which were not cross-linked was detected by ELISA. The results show that the degree of cross-linking of #10 and #16 with VEGF was greater than that of competitor AK112 analog, which was mutually supported by the results of structural biology simulations. This property will enable VEGF to be enriched locally in the tumor through its high local expression, demonstrating the differentiated characteristics and therapeutic potential of the #10 and #16 bispecific antibodies.

### Example 13: Efficacy in Animal Model

In order to evaluate the efficacy of the test antibodies in the mouse model, Balb/c mice transformed with human PD-1 were selected and inoculated with wild-type CT26 tumor cells (colon cancer cells). Since neither of Bevacizumab and AK112 analog bound to mouse VEGF, these two groups were not added. VEGF Trap (a fusion protein composed of the second domain of VEGFR1, the third domain of VEGFR2 and human IgGFc) that can bind to human VEGF and mouse VEGF was selected as a control. A total of 6 groups were designed. The experimental design, administration amount, and route of administration are shown in Table 17.

**Table 17 Design of efficacy experiments for animal model**

| Test group | Drug | Number of mice | Administration amount (mg/kg) | Administration frequency | Route of administr ation |
|---|---|---|---|---|---|
| 1 | Blank vector (PBS) | 8 | 0 | D0, D4, D7, D11 | i.p. |
| 2 | Opdivo | 8 | 7.5 | D0, D4, D7, D11 | i.p. |
| 3 | VEGF-Trap | 8 | 5 | D0, D4, D7, D11 | i.p. |
| 4 | Opdivo+VEGF-Trap | 8 | 7.5+5 | D0, D4, D7, D11 | i.p. |
| 5 | #10 | 8 | 8.8 | D0, D4, D7, D11 | i.p. |
| 6 | #16 | 8 | 8.9 | D0, D4, D7, D11 | i.p. |

As shown in FIG. 8, the tumor inhibition rates of tumor-bearing mice treated with #10 and #16 were 57% and 62%, respectively, which were higher than the tumor inhibition rate of (20%) the Opdivo monotherapy group and the tumor inhibition rate (45%) of the VEGF-Trap monotherapy group, and comparable to the tumor inhibition rate (56%) of the Opdivo and VEGF-Trap combined therapy group. On day 14, the average tumor volume of the #10 and 16 treatment groups was slightly smaller than that of the combined therapy group. Additionally, the weight changes of the mice were consistent with the trend of tumor growth. On day 14, the weight changes of #10 and #16 were within 10%. It can be seen that both #10 and #16 had good efficacy and safety.

### Example 14 Study on the synergistic effect of #10 in immune-reconstituted human melanoma subcutaneous transplantation model

In order to evaluate the synergistic effect of #10 in the immune-reconstituted human melanoma model, immunodeficient mice B-NDG were inoculated with A375 tumor cells (melanoma cells) transformed with human PD-L1, and inoculated with human peripheral blood mononuclear cells (huPBMC) to reconstruct the immune system of tumor-bearing mice. The anti-PD-1 drug Opdivo (O drug), the anti-VEGF drug Bevacizumab, and #48 were selected as single-target controls, and AK112 analog (AK 112 analog, in-house expressed and prepared) was selected as the control drug of #10. A total of 8 groups were designed. The experimental design, administration amount, and route of administration are shown in Table 18.

**Table 18 Experimental design of synergistic study in immune-reconstituted human melanoma subcutaneous transplantation model**

| Test group | Drug | Number of mice | Administration amount (mg/kg) | Administration frequency | Route of administration |
|---|---|---|---|---|---|
| 1 | Blank vector | 9 | -- | D0, D3, D7, D10, D14, D17 | i.v. |
| 2 | #10 | 9 | 5.7 | D0, D3, D7, D10, D14, D17 | i.v. |
| 3 | Bevacizumab | 9 | 5 | D0, D3, D7, D10, D14, D17 | i.v. |
| 4 | #48 | 9 | 2.6 | D0, D3, D7, D10, D14, D17 | i.v. |
| 5 | Opdivo | 9 | 4.9 | D0, D3, D7, D10, D14, D17 | i.v. |
| 6 | Bevacizumab + Opdivo | 9 | 5+4.9 | D0, D3, D7, D10, D14, D17 | i.v. |
| 7 | #48 + Opdivo | 9 | 2.6+4.9 | D0, D3, D7, D10, D14, D17 | i.v. |
| 8 | AK112 analog | 9 | 6.8 | D0, D3, D7, D10, D14, D17 | i.v. |

The detection was performed on day 21. In this experimental system, the Opdivo monotherapy group had no anti-tumor effect, the tumor inhibition rate of #10 was TGI=73%, showing a tumor inhibition level significantly higher than that of the Bevacizumab monotherapy group (TGI=53%), #48 monotherapy group (TGI=57%), and Opdivo and #48 combined treatment group (TGI=50%), and superior to the combined treatment group of Opdivo and Bevacizumab (TGI=68%, based on which, the tumor inhibition rate of #10 was increased by 7%) and AK112 analog (TGI=64%, based on which, the relative tumor inhibition rate of #10 was increased by 14%). The results show that the bispecific antibodies of the present disclosure produced a synergistic effect and had significant advantages over the control molecules.

Only some of the embodiments of the present disclosure are disclosed above, which do not limit the present disclosure in any form. It can be understood by those of ordinary skill in the art that the present disclosure is not limited to each specific embodiment. For those of ordinary skill in the art, several improvements and modifications can be made to the present disclosure without departing from the principle of the present disclosure, and these improvements and modifications also fall within the scope of protection of the claims of the present disclosure.

## Claims

1. A bispecific antibody targeting programmed death receptor-1 (PD-1) and vascular endothelial growth factor (VEGF), comprising:
(a) a first binding functional region targeting PD-1; and
(b) a second binding functional region targeting VEGF;
wherein,
the first binding functional region targeting PD-1 is an anti-PD-1 antibody or an antigen-binding fragment thereof; and
the second binding functional region targeting VEGF comprises a variable domain of heavy chain of heavy-chain antibody (VHH) domain of complementarity-determining regions (CDRs) 1-3, wherein CDR1 comprises an amino acid sequence set forth in SEQ ID NO: 1, CDR2 comprises an amino acid sequence set forth in SEQ ID NO: 2 with or without one or two amino acid mutations, and CDR3 comprises an amino acid sequence set forth in SEQ ID NO: 3.

2. The bispecific antibody according to claim 1, wherein the amino acid mutation in CDR2 is at position 58 and/or position 65.

3. The bispecific antibody according to claim 2, wherein the amino acid mutation in CDR2 is N58Y and/or D65G mutation.

4. The bispecific antibody according to claim 1, wherein the VHH domain is a humanized VHH domain.

5. The bispecific antibody according to claim 1 or 3, wherein the second binding functional region targeting VEGF further comprises:
1) a first framework region domain FR1 comprising an amino acid sequence set forth in SEQ ID NO: 4 with or without one or two amino acid mutations; and/or
2) a second framework region domain FR2 comprising an amino acid sequence set forth in SEQ ID NO: 5 with or without one amino acid mutation; and/or
3) a third framework region domain FR3 comprising an amino acid sequence set forth in SEQ ID NO: 6 with or without 1-5 amino acid mutations; and/or
4) a fourth framework region domain FR4 comprising an amino acid sequence set forth in SEQ ID NO: 7 with or without one amino acid mutation.

6. The bispecific antibody according to claim 5, wherein the one or two amino acid mutations in FR1 are at position 1 and/or position 5; preferably Q1E and/or Q5L mutations.

7. The bispecific antibody according to claim 6, wherein the one amino acid mutation in FR2 is at position 49; preferably A49S mutation.

8. The bispecific antibody according to claim 7, wherein the 1-5 amino acid mutations in FR3 are at positions selected from the group consisting of positions 74, 82B, 83, 84, 89, and a combination thereof; preferably, the 1-5 amino acid mutations are selected from the group consisting of D74S, V(82B)S, K83R, P84A, M89V and a combination thereof.

9. The bispecific antibody according to claim 8, wherein the one amino acid mutation in FR4 is at position 108; preferably Q108L.

10. The bispecific antibody according to any one of claims 5 to 9, wherein the second binding functional region targeting VEGF further comprises:
1) a first framework region domain FR1 comprising an amino acid sequence set forth in SEQ ID NO: 4 or SEQ ID NO: 9; and/or
2) a second framework region domain FR2 comprising an amino acid sequence set forth in SEQ ID NO: 5 or SEQ ID NO: 10; and/or
3) a third framework region domain FR3 comprising an amino acid sequence set forth in SEQ ID NO: 6 or SEQ ID NO: 11; and/or
4) a fourth framework region domain FR4 comprising an amino acid sequence set forth in SEQ ID NO: 7 or SEQ ID NO: 12.

11. The bispecific antibody according to claim 10, wherein the second binding functional region targeting VEGF comprises:
1) a framework region combination of FR1 comprising an amino acid sequence set forth in SEQ ID NO: 4, FR2 comprising an amino acid sequence set forth in SEQ ID NO: 5, FR3 comprising an amino acid sequence set forth in SEQ ID NO: 6, and FR4 comprising an amino acid sequence set forth in SEQ ID NO: 7; or
2) a framework region combination of FR1 comprising an amino acid sequence set forth in SEQ ID NO: 9, FR2 comprising an amino acid sequence set forth in SEQ ID NO: 10, FR3 comprising an amino acid sequence set forth in SEQ ID NO: 11, and FR4 comprising an amino acid sequence set forth in SEQ ID NO: 12; or
3) a framework region combination of FR1 comprising an amino acid sequence set forth in SEQ ID NO: 9, FR2 comprising an amino acid sequence set forth in SEQ ID NO: 5, FR3 comprising an amino acid sequence set forth in SEQ ID NO: 11, and FR4 comprising an amino acid sequence set forth in SEQ ID NO: 12.

12. The bispecific antibody according to claim 1, wherein the second binding functional region targeting VEGF comprises an amino acid sequence set forth in SEQ ID NO: 8 with or without 1-11 amino acid mutations.

13. The bispecific antibody according to claim 12, wherein the amino acid mutation is at a position selected from the group consisting of positions 1, 5, 49, 58, 65, 74, 82B, 83, 84, 89, 108, and a combination thereof.

14. The bispecific antibody according to claim 13, wherein the amino acid mutation is selected from the group consisting of Q1E, Q5L, A49S, N58Y, D65G, D74S, V(82B)S, K83R, P84A, M89V, Q108L and a combination thereof.

15. The bispecific antibody according to claim 14, wherein the amino acid mutation is a mutation combination of
1) Q1E+Q5L+A49S+N58Y+D65G+D74S+V(82B)S+K83R+P84A+M89V+Q108L; or
2) Q1E+Q5L+N58Y+D65G+D74S+V(82B)S+K83R+P84A+M89V+Q108L.

16. The bispecific antibody according to claim 1, wherein the second binding functional region targeting VEGF comprises an amino acid sequence set forth in SEQ ID NO: 8, SEQ ID NO: 13 or SEQ ID NO: 14.

17. The bispecific antibody according to any one of claims 1 to 16, wherein the anti-PD-1 antibody or the antigen-binding fragment thereof is selected from the group consisting of a humanized antibody or an antigen-binding fragment thereof, a chimeric antibody or an antigen-binding fragment thereof, and a human antibody or an antigen-binding fragment thereof.

18. The bispecific antibody according to claim 17, wherein the anti-PD-1 antibody or the antigen-binding fragment thereof is an IgG antibody.

19. The bispecific antibody according to claim 18, wherein the anti-PD-1 antibody or the antigen-binding fragment thereof is IgG1, IgG2, or IgG4.

20. The bispecific antibody according to claim 19, wherein the first binding functional region targeting PD-1 comprises an amino acid sequence of light chain and heavy chain CDRs identical to that of nivolumab antibody or pembrolizumab antibody.

21. The bispecific antibody according to claim 20, wherein the first binding functional region targeting PD-1 comprises an amino acid sequence of heavy chain variable region identical to nivolumab antibody or pembrolizumab antibody.

22. The bispecific antibody according to claim 21, wherein the first binding functional region targeting PD-1 comprises an amino acid sequence of light chain variable region identical to nivolumab antibody or pembrolizumab antibody.

23. The bispecific antibody according to claim 22, wherein the first binding functional region targeting PD-1 comprises a heavy chain moiety, wherein the heavy chain moiety further comprises a domain selected from the group consisting of a heavy chain constant region CH1, a hinge region, a heavy chain constant region CH2, a heavy chain constant region CH3, and a combination thereof.

24. The bispecific antibody according to claim 23, wherein the first binding functional region targeting PD-1 further comprises a light chain constant region.

25. The bispecific antibody according to claim 24, wherein the anti-PD-1 antibody comprises a heavy chain moiety and a light chain moiety, wherein the heavy chain moiety comprises, from N-terminus to C-terminus, a heavy chain variable region, a heavy chain constant region CH1, a hinge region, a heavy chain constant region CH2, and a heavy chain constant region CH3, and the light chain moiety comprises, from N-terminus to C-terminus, a light chain variable region and a light chain constant region.

26. The bispecific antibody according to claim 25, wherein the anti-PD-1 antibody comprises an Fc domain, wherein the Fc domain is an IgG1 Fc domain or a silent IgG1 mutation thereof.

27. The bispecific antibody according to claim 26, wherein the Fc domain comprises an amino acid sequence set forth in SEQ ID NO: 15 or at least 90%, 91%, 92%, 93%, 94%, 95%, 96%, 97%, 98%, 99% or more identical to an amino acid sequence set forth in SEQ ID NO: 15.

28. The bispecific antibody according to claim 27, wherein the first binding functional region targeting PD-1 comprises a light chain and heavy chain combination of
1) a heavy chain comprising an amino acid sequence set forth in SEQ ID NO: 16, and/or a light chain comprising an amino acid sequence set forth in SEQ ID NO: 17; or
2) a heavy chain comprising an amino acid sequence set forth in SEQ ID NO: 18, and/or a light chain comprising an amino acid sequence set forth in SEQ ID NO: 19.

29. The bispecific antibody according to claim 28, wherein the second binding functional region is linked to the C-terminus or N-terminus of the first binding functional region.

30. The bispecific antibody according to claim 29, wherein the VHH domain is directly or via a peptide linker linked to the C-terminus or N-terminus of the heavy chain moiety of the antibody or the antigen-binding fragment thereof.

31. The bispecific antibody according to claim 30, wherein the peptide linker is a flexible peptide linker.

32. The bispecific antibody according to claim 31, wherein the peptide linker comprises one or more amino acids.

33. The bispecific antibody according to claim 32, wherein the peptide linker comprises at least 5 amino acids.

34. The bispecific antibody according to claim 33, wherein the peptide linker comprises an amino acid sequence of (GGGGS)ₙ, wherein n is 1, 2, 3 or 4.

35. The bispecific antibody according to claim 29 or 30, wherein the bispecific antibody is a bivalent, trivalent or tetravalent bispecific antibody.

36. The bispecific antibody according to claim 1, wherein the bispecific antibody comprises a combination of a heavy chain set forth in SEQ ID NO: 20 and a light chain set forth in SEQ ID NO: 21, or comprises a combination of a heavy chain set forth in SEQ ID NO: 22 and a light chain set forth in SEQ ID NO: 23.

37. A VHH domain targeting VEGF, wherein the VHH domain comprises CDRs 1-3, wherein CDR1 comprises an amino acid sequence set forth in SEQ ID NO: 1, CDR2 comprises an amino acid sequence set forth in SEQ ID NO: 2 with or without one or two amino acid mutations, and CDR3 comprises an amino acid sequence set forth in SEQ ID NO: 3.

38. The VHH domain according to claim 37, wherein the amino acid mutation in CDR2 is at position 58 and/or position 65.

39. The VHH domain according to claim 38, wherein the amino acid mutation in CDR2 is N58Y and/or D65G mutation.

40. The VHH domain according to claim 39, wherein the VHH domain is a humanized VHH domain.

41. The VHH domain according to claim 40, wherein the VHH domain further comprises:
1) a first framework region domain FR1 comprising an amino acid sequence set forth in SEQ ID NO: 4 with or without one or two amino acid mutations; and/or
2) a second framework region domain FR2 comprising an amino acid sequence set forth in SEQ ID NO: 5 with or without one amino acid mutation; and/or
3) a third framework region domain FR3 comprising an amino acid sequence set forth in SEQ ID NO: 6 with or without 1-5 amino acid mutations; and/or
4) a fourth framework region domain FR4 comprising an amino acid sequence set forth in SEQ ID NO: 7 with or without one amino acid mutation.

42. The VHH domain according to claim 41, wherein the one or two amino acid mutations in FR1 are at position 1 and/or position 5; preferably Q1E and/or Q5L mutations; further, the one amino acid mutation in FR2 is at position 49; preferably A49S mutation.

43. The VHH domain according to claim 42, wherein the 1-5 amino acid mutations in FR3 are at positions selected from the group consisting of positions 74, 82B, 83, 84, 89, and a combination thereof; preferably, the 1-5 amino acid mutations are selected from the group consisting of D74S, V(82B)S, K83R, P84A, M89V and a combination thereof.

44. The VHH domain according to claim 43, wherein the one amino acid mutation in FR4 is at position 108; preferably Q108L.

45. The VHH domain according to claim 44, wherein the VHH domain further comprises:
1) a first framework region domain FR1 comprising an amino acid sequence set forth in SEQ ID NO: 4 or SEQ ID NO: 9; and/or
2) a second framework region domain FR2 comprising an amino acid sequence set forth in SEQ ID NO: 5 or SEQ ID NO: 10; and/or
3) a third framework region domain FR3 comprising an amino acid sequence set forth in SEQ ID NO: 6 or SEQ ID NO: 11; and/or
4) a fourth framework region domain FR4 comprising an amino acid sequence set forth in SEQ ID NO: 7 or SEQ ID NO: 12.

46. The VHH domain according to claim 45, wherein the VHH domain comprises:
1) a framework region combination of FR1 comprising an amino acid sequence set forth in SEQ ID NO: 4, FR2 comprising an amino acid sequence set forth in SEQ ID NO: 5, FR3 comprising an amino acid sequence set forth in SEQ ID NO: 6, and FR4 comprising an amino acid sequence set forth in SEQ ID NO: 7;
2) a framework region combination of FR1 comprising an amino acid sequence set forth in SEQ ID NO: 9, FR2 comprising an amino acid sequence set forth in SEQ ID NO: 10, FR3 comprising an amino acid sequence set forth in SEQ ID NO: 11, and FR4 comprising an amino acid sequence set forth in SEQ ID NO: 12; or
3) a framework region combination of FR1 comprising an amino acid sequence set forth in SEQ ID NO: 9, FR2 comprising an amino acid sequence set forth in SEQ ID NO: 5, FR3 comprising an amino acid sequence set forth in SEQ ID NO: 11, and FR4 comprising an amino acid sequence set forth in SEQ ID NO: 12.

47. The VHH domain according to claim 37, wherein the VHH domain comprises an amino acid sequence set forth in SEQ ID NO: 8 with or without 1-11 amino acid mutations.

48. The VHH domain according to claim 47, wherein the amino acid mutation is at a position selected from the group consisting of positions 1, 5, 49, 58, 65, 74, 82B, 83, 84, 89, 108, and a combination thereof.

49. The VHH domain according to claim 48, wherein the amino acid mutation is selected from the group consisting of Q1E, Q5L, A49S, N58Y, D65G, D74S, V(82B)S, K83R, P84A, M89V, Q108L and a combination thereof.

50. The VHH domain according to claim 49, wherein the mutation is a mutation combination of
1) QIE+Q5L+A49S+N58Y+D65G+D74S+V(82B)S+K83R+P84A+M89V+Q108L; or
2) Q1E+Q5L+N58Y+D65G+D74S+V(82B)S+K83R+P84A+M89V+Q108L.

51. The VHH domain according to claim 50, wherein the VHH domain comprises an amino acid sequence set forth in SEQ ID NO: 8, SEQ ID NO: 13 or SEQ ID NO: 14.

52. Use of the VHH domain according to any one of claims 37 to 51 in the construction of a recombinant protein.

53. A recombinant protein comprising the VHH domain according to any one of claims 37-51.

54. The recombinant protein according to claim 53, wherein the recombinant protein is selected from the group consisting of a bispecific antibody, a multispecific antibody, and an antibody-drug conjugate.

55. A polynucleotide encoding the bispecific antibody according to any one of claims 1 to 36, the VHH domain according to any one of claims 37 to 51, the recombinant protein according to claim 53, or the multispecific antibody according to claim 54.

56. An expression vector comprising the polynucleotide according to claim 55.

57. A host cell comprising the expression vector according to claim 56 or integrated with the polynucleotide according to claim 55 into the genome.

58. A pharmaceutical composition comprising the bispecific antibody according to any one of claims 1 to 36, the VHH domain according to any one of claims 37 to 51, the recombinant protein according to claim 53, or the multispecific antibody according to claim 54, and a pharmaceutically acceptable carrier.

59. Use of the bispecific antibody according to any one of claims 1 to 36, the VHH domain according to any one of claims 37 to 51, the recombinant protein according to claim 53, or the multispecific antibody according to claim 54 in the manufacture of a medicament for treating cancer.

60. A method for treating cancer, comprising administering to a subject in need thereof an effective amount of the bispecific antibody according to any one of claims 1 to 36, the VHH domain according to any one of claims 37 to 51, the recombinant protein according to claim 53, or the multispecific antibody according to claim 54.

61. The use according to claim 59 or the method according to claim 60, wherein the cancer is colorectal cancer.
